# EUROPÄISCHE PATENTSCHRIFT

(11) **EP 2 099 824 B1**
(45) Veröffentlichungstag und Bekanntmachung des Hinweises auf die Patenterteilung: **03.09.2014**
(21) Anmeldenummer: 07846571.3
(22) Anmeldetag: 13.11.2007
(51) Int. Cl.: A61K 39/07, C07H 15/04, C08B 37/00, G01N 33/569

(54) **ENTEROCOCCUS FAECALIS- UND/ODER ENTEROCOCCUS FAECIUM-ANTIGEN**
ENTEROCOCCUS FAECALIS AND/OR ENTEROCOCCUS FAECIUM ANTIGEN
ANTIGÈNE ENTEROCOCCUS FAECALIS ET/OU ENTEROCOCCUS FAECIUM

(30) Priorität: 13.11.2006 DE 102006053385
(43) Veröffentlichungstag der Anmeldung: 16.09.2009
(73) Patentinhaber: Universitätsklinikum Freiburg, 79106 Freiburg (DE)
(72) Erfinder: HÜBNER, Johannes, 79106 Freiburg (DE); HOLST, Otto, 23843 Bad Oldesloe (DE); THEILACKER, Christian, 79104 Freiburg (DE); KACZYNSKI, Zbigniew, 80-180 Gdansk (PL)
(74) Vertreter: Krauss, Jan
(86) Internationale Anmeldenummer: PCT/EP2007/009813
(87) Internationale Veröffentlichungsnummer: WO 2008/058706

(56) Entgegenhaltungen:
- WO-A-99/08705
- THEILACKER C ET AL: "OPSONIC ANTIBODIES TO ENTEROCOCCUS FAECALIS STRAIN 12030 ARE DIRECTED AGAINST LIPTEICHOIC ACID" INFECTION AND IMMUNITY, AMERICAN SOCIETY FOR MICROBIOLOGY. WASHINGTON, Bd. 74, Nr. 10, 1. Oktober 2006 (2006-10-01), Seiten 5703-5712, XP009074456 ISSN: 0019-9567
- HUFNAGEL, M. ET AL.: "Serological and genetic diversity of capsular polysaccharides in Enterococcus faecalis" J. CLIN. MICROBIOL., Bd. 42, Nr. 6, 2004, Seiten 2548-2557, XP002496176
- HSU CAROLYN T ET AL: "Immunochemical characterization of polysaccharide antigens from six clinical strains of Enterococci" BMC MICROBIOLOGY, BIOMED CENTRAL, LONDON, GB, Bd. 6, Nr. 1, 12. Juli 2006 (2006-07-12), Seite 62, XP021014894 ISSN: 1471-2180 in der Anmeldung erwähnt

## Beschreibung

Die vorliegende Erfindung betrifft allgemein das Gebiet der Detektion und Prävention von Infektionskrankheiten, die durch *Enterococcus faecalis* und/oder *Enterococcus faecium* verursacht werden.

Insbesondere betrifft die vorliegende Erfindung ein Antigen gemäß Anspruch 1, Antikörper gemäß Anspruch 8 und 26, Zusammensetzungen gemäß Anspruch 9, Verfahren gemäß Anspruch 13 und 27, ein Kit gemäß Anspruch 19 und eine Verwendung gemäß Anspruch 24.

Nosokomiale Infektionen (Krankenhausinfektionen) sind durch Mikroorganismen hervorgerufene Infektion, die in kausalem Zusammenhang zu einem Krankenhausaufenthalt stehen.

In den USA stieg der Anteil an Nosokomialinfektionen von 1975-1995 um 36% an, so dass im Jahre 1995 etwa 10 von 1000 Patienten von solch einer Infektion betroffen waren.

Nosokomialinfektionen sind so allein in den USA im Jahr für 44.000 - 98.000 Todesfälle verantwortlich.

Krankenhausinfektionen sind verantwortlich für einen Grossteil aller im Hospital auftretenden Komplikationen, und deren Vermeidung ist wesentlich für die Qualität der medizinischen und krankenpflegerischen Versorgung der Patienten.

Sie sind daher ein ernstzunehmendes Problem eines jeden Krankenhauses, da bekanntlich das oberste Ziel aller qualitätsorientierten Handlungen in der Krankenhausbehandlung der gesunde Patient ist.

Nosokomiale Infektionen belasten nicht nur den Patienten selbst durch zusätzliche Beschwerden, sie verlängern meist auch den Krankenhausaufenthalt - im Durchschnitt um etwa 4 Tage - und tragen somit zur Überbelastung der Krankenhäuser und des Gesundheitssystems bei. In den USA verursachen nosokomiale Infektionen pro Jahr Kosten in Höhe von 17 bis 29 Milliarden US-Dollar.

Trotz des unbestreitbaren medizinischen Fortschritts wird für die Zukunft erwartet, dass die Häufigkeit nosokomialer Infektionen eher zunehmen wird.

Unter anderen werden die folgenden Faktoren für diese Entwicklung verantwortlich gemacht:
- Die Anzahl der Patienten mit hohem Alter steigt,
- Patienten mit geschwächter körpereigener Infektionsabwehr werden in den Krankenhäusern in zunehmender Anzahl behandelt,
- kompliziertere und schwierigere Operationen werden aufgrund der Fortschritte in der operativen Technik durchgeführt,
- komplizierte apparative, invasive Maßnahmen mit erhöhtem Infektionsrisiko können zunehmend durchgeführt werden,
- therapeutische Maßnahmen, die die Abwehrkraft herabsetzen, werden zunehmend durchgeführt, und
- Antibiotika, insbesondere Breitspektrum-Antibiotika, werden vermehrt eingesetzt was zur Zunahme von multiresistenten Mikroorganismen führt.

Jüngere Studien, die in diesem Zusammenhang durchgeführt wurden (Am. J. Infect. Control., 32:470-485, 2004) haben gezeigt, dass bis zu 25% der *Enterococcus faecium*-Isolate aus Krankenhäusern gegen glykopeptidische Antibiotika resistent sind.

Glykopeptidische Antibiotika greifen die Zellwand an und fügen sich direkt in die Struktur der Zellwand ein, wodurch Löcher entstehen und Wasser eindringen kann. Ein Beispiel für glykopeptidische Antibiotika ist das Vancomycin.

DiazGranados, C.A., et al. haben kürzlich gezeigt, dass Vancomycin-resistente Enterokokken eine erhebliche Krankhaftigkeit und Sterblichkeit insbesondere bei Patienten verursachen, die ohnehin schon ein geschwächtes Immunsystem haben (Clin. Infect. Dis., 41:327-333, 2005).

Es gibt daher ein fortwährendes Interesse, immuntherapeutische Herangehensweisen zu entwickeln, die es erlauben, solche Infektionen zu verhindern oder zumindest zu kontrollieren.

Es ist im Stand der Technik zwar grundsätzlich bekannt, Kohlenhydrat-Antigene bei der Entwicklung von Impfstoffen zu verwenden (Ada, G., et al., Clin. Microbiol. Infect., 9: 79-85, 2003), jedoch weiß man bisher nur wenig über Zellwandassoziierte und kapsuläre Polysaccharide bei *Enterococcus faecalis.*

Bis heute wurden vier Kohlenhydrat-Antigene beschrieben (Hancock, et al., Proc. Natl. Acad. Sci USA, 99:1574-1579, 2002; Hsu et al., BMC Microbiol., 6:62, 2006; Pazur et al., J. Biol. Chem., 248: 279-284, 1973; Wang et al., Carbohydr. Res. 316: 155-160, 1999; Xu et al., Infect. Immun., 65:4207-4215, 1997; Xu et al., Infect. Immun. 66:4313-4323, 1998; Xu et al., Infect. Immun., 68:815-823, 2000), aber eine vollständige Strukturanalyse liegt nur für eines von ihnen vor. Es wird ferner ein Kohlenhydrat-Antigen beschrieben, das an der Zelloberfläche des *Enterococcus faecalis-Stammes* FA2-2 exponiert ist und das aus Glucose, Galactose und Glycerinphosphat besteht.

WO 99/08705 und Theilacker et al. (in Infect. Immun. 2006, 5703-5712 (2006)) beschreiben aus *Enterococcus faecalis* isolierbare Polysaccharidantigene, die sich in ihrer Struktur jedoch von dem in der vorliegenden Anmeldung beschriebenen Polysaccharidantigen unterscheiden.

Hufnagel et al. (in J. Clin. Microbiol. 42, 2548-2557 (2004)) beschreiben Sera mit Antikörpern gegen ganze Bakterienzellen von *E. faecalis* Typ 5. Es werden jedoch nur allgemein Antikörper gegen alle möglichen Kapsel-Antigene (polyklonale Antiseren, "type specific sera") beschrieben. Die erwähnten "Maekawa-Stämme" der Publikation sind zudem ein Gemisch von Typen 2 und 5, somit sind die Antiseren nicht spezifisch.

Um der Problematik Herr zu werden, die durch das Auftreten von multiresistenten *Enterococcus faecalis-Stämmen* und den nahe verwandten *Enterococcus faecium-Stämmen* verursacht wird, besteht ein erheblicher Bedarf im Stand der Technik, Antigene zu charakterisieren, die auf der Oberfläche von *Enterococcus faecalis* und/oder *Enterococcus faecium* exponiert sind, um diese zur Überwindung der oben beschriebenen Probleme zu verwenden.

Es ist daher eine Aufgabe der vorliegenden Erfindung, bisher unbekannte Antigene von *Enterococcus faecalis* und/oder *Enterococcus faecium* zur Verfügung zu stellen.

Es ist eine weitere Aufgabe der vorliegenden Erfindung, Antikörper gegen diese bisher unbekannten Antigene von *Enterococcus faecalis* und/oder *Enterococcus faecium* zur Verfügung zu stellen.

Es ist eine weitere Aufgabe der vorliegenden Erfindung, eine Zusammensetzung zur Verfügung zu stellen, die wenigstens ein Antigen von *Enterococcus faecalis* und/oder *Enterococcus faecium* oder einen Antikörper dagegen umfasst, die bevorzugt zur aktiven bzw. passiven Immunisierung gegen *Enterococcus faecalis* und/oder *Enterococcus faecium-Infektionen* geeignet ist.

Es ist eine weitere Aufgabe der vorliegenden Erfindung ein Verfahren zur Verfügung zu stellen, das die Detektion des bisher unbekannten Antigens in einer Probe ermöglicht.

Es ist eine weitere Aufgabe der vorliegenden Erfindung ein Verfahren zur Verfügung zu stellen, das die Detektion von Antikörpern gegen das bisher unbekannte Antigen in einer Probe ermöglicht.

Es ist eine weitere Aufgabe der vorliegenden Erfindung, ein Kit zur Verfügung zu stellen, das die Detektion des bisher unbekannten Antigens oder von Antikörpern gegen dieses Antigen ermöglicht.

Es ist eine weitere Aufgabe der vorliegenden Erfindung eine Verwendungsmöglichkeit des bisher unbekannten Antigens zur Detektion oder Herstellung entsprechender Antikörper oder Antikörperfragmente sowie ein entsprechendes Verfahren zur Verfügung zu stellen.

Diese Aufgaben der vorliegenden Erfindung werden durch das Antigen gemäß Anspruch 1, Antikörper gemäß Anspruch 8 und 26, Zusammensetzungen gemäß Anspruch 9, Verfahren gemäß Anspruch 13 und 27, ein Kit gemäß Anspruch 19 und eine Verwendung gemäß Anspruch 24 gelöst.

Insbesondere stellt die vorliegende Erfindung ein *Enterococcus faecalis* und/oder *Enterococcus faecium*-Antigen zur Verfügung, das dadurch gekennzeichnet ist, dass es wenigstens eine aus einer Furanose Gal und aus einer Pyranose Glc bestehende Disaccharidstruktur aufweist, wobei die Disaccharidstruktur die folgende Formel aufweist: 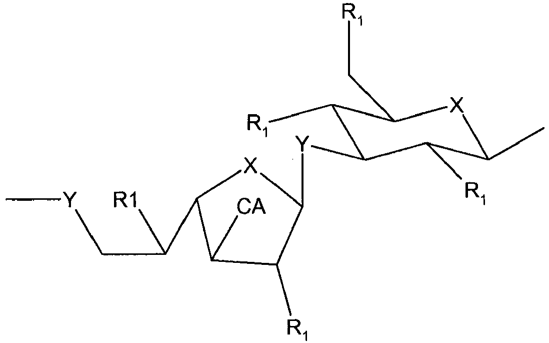
wobei R₁ unabhängig voneinander OH, OAc, OCₙHₘ, OFo, wobei Fo Formyl, m = 2n + 1 und n eine natürliche Zahl von 1 bis 10 ist,
X = O,
Y = O und
CA unabhängig voneinander ein C₁-C₆-Acyl- oder ein Hydroxyacylrest ist.

Vorzugsweise liegen in dieser Einheit die beiden Zucker in D-Konfiguration vor.

Besonders bevorzugt ist das Antigen der vorliegenden Erfindung dadurch gekennzeichnet, dass das aus einer Furanose Gal und aus einer Pyranose Glc bestehende Disaccharid eine Struktur hat, die ausgewählt ist aus den folgenden:
**→-u]-D-Galf-|1→-z| -D-Glcp- [1→- ,**
**→-u]-D-Galf- |1→-z|-D-Glcf- [1→- ,**
**→-u]-D-Galp-|1→-z]-D-Glcp- [1→- oder**
**→-u]-D-Galp- |1→-z|-D-Glcf- [1→-,**
wobei v und z jeweils 1, 2, 3, 4, 5 oder 6 sind.

Weiter bevorzugt ist R₁ = OH, X = O, Y = O und CA = Lactyl.

Das Molekulargewicht der Antigene der vorliegenden Erfindung ist grundsätzlich unkritisch und daher nicht weiter eingeschränkt. Typischerweise haben die Antigene der vorliegenden Erfindung jedoch Molekulargewichte von ca. 1000 - 200000 Da, mehr bevorzugt von ca. 50000-150000 Da, bevorzugt von ca. 100000 Da. Zu kleine Molekulargewichte können möglicherweise zur Folge haben, dass das Antigen aufgrund der wenigen intramolekularen Wechselwirkungen keine strukturell stabilen Epitope ausbilden kann, während zu hohe Molekulargewichte zur Folge haben, dass solche Antigen oft schwierig zu synthetisieren sind und deren Haltbarkeit eingeschränkt ist.

Für das Antigen der vorliegenden Erfindung ist es grundsätzlich ausreichend, wenn es die Einheit aus Anspruch 1 mindestens 1 mal enthält. Allerdings kann die Antigenizität des Antigens der vorliegenden Erfindung dadurch gesteigert werden, dass die Einheit pro Antigen mindestens 5 mal, bevorzugt mindestens 10 mal, mehr bevorzugt mindestens 100 mal, besonders bevorzugt mindestens 1000 mal vorkommt.
Für verschiedene analytische Anwendungen des Antigens der vorliegenden Erfindung kann es ferner bevorzugt sein, wenn das Antigen immobilisiert ist. Hierdurch kann die Präsenz des Antigens örtlich beschränkt werden, so dass beispielsweise im Rahmen eines Arrays viele unterschiedliche Proben gleichzeitig und doch getrennt voneinander mit dem Antikörper in Kontakt gebracht und analysiert werden. Auch bei affinitätsbasierenden Reinigungsverfahren, beispielsweise für entsprechende Antikörper, sind immobilisierte Antigen nützlich.

Daher ist in einer bevorzugten Ausführungsform der Erfindung das Antigen an einen Träger, vorzugsweise einen Immunträger gebunden.

Grundsätzlich kann die Immobilisierung durch jegliche Art von Wechselwirkungen erfolgen, wie bspw. Van-der-Waals-Kräfte, ionische Interaktionen, Dipol-Dipol-Wechselwirkungen, Wasserstoffbrückenbindungen oder hydrophobe Interaktionen. Bevorzugt ist jedoch, dass die Bindung an den Träger kovalenter Natur ist.

Bevorzugt wird das Antigen der vorliegenden Erfindung in Form einer Zusammensetzung zur Verfügung gestellt. Gemäss einer bevorzugten Ausführungsform umfasst eine solche Zusammensetzung mindestens einen pharmazeutisch akzeptablen Träger. Eine Trägersubstanz ist hierbei jede Substanz, an die das Antigen angelagert und/oder physikalisch gebunden werden kann. Beispielsweise kann so das Antigen, das sich sonst nur schwer dosieren lässt, an einen leichter zu dosierenden Träger gebunden werden. Beispiele für solche Träger sind Stärke und Maltodextrin. Pharmazeutisch akzeptabel bedeutet hierbei, dass der verwendete Träger nichttoxisch ist und mit der Wirkung des Antigens nicht interferiert.

Das Antigen der vorliegenden Erfindung kann als eine vorbeugende Massnahme im Rahmen einer aktiven Impfung gegen durch Enterococcus faecalis und/oder Enterococcus faecium ausgelöste Infektionskrankheiten einem Patienten verabreicht werden. Ziel einer solchen Impfung ist es, das körpereigene Immunsystem selbst zur Bildung von spezifischen Antikörpern anzuregen und so eine spezifische Immunität gegen Enterococcus faecalis und/oder Enterococcus faecium zu bewirken. Als Patient kommen daher sämtliche Lebewesen in Betracht, die über ein Immunsystem verzügen. Von besonderer Wichtigkeit sind jedoch Säugetiere, wie beispielsweise Menschen, nicht-humane Primaten, Hunde, Katzen, Schweine, Kühe, Pferde, Ziegen und Schafe und Vögel, wie beispielsweise Hühner, Enten, Gänse, Truthähne oder Strausse.

Insofern stellt in einer bevorzugten Ausführungsform das Antigen der vorliegenden Erfindung ein Vakzin gegen Enterococcus faecalis und/oder Enterococcus faecium dar.

Ebenfalls Gegenstand der vorliegenden Erfindung ist ein monoklonaler Antikörper, der gegen das oben beschriebene Antigen gerichtet ist.

Antikörper bestehen aus zwei identischen schweren Ketten (heavy chains, H) und zwei identischen leichten Ketten (light chains, L), die durch kovalente Disulfidbrücken zu einer Ypsi-Ion-förmigen Struktur miteinander verknüpft sind. Die beiden Leichtketten sind je nach Organismus und ImmunglobulinSubklasse entweder vom Typ kappa oder lambda und bilden zusammen mit den oberhalb der Gelenkregion (hinge region) liegenden Anteil der schweren Ketten das antigenbindende Fragment Fab, welches enzymatisch von dem darunterliegenden kristallinen Fragment Fc abgespalten werden kann.

Die ausgesprochene Variabilität der Antikörperbindungsstellen (abgekürzt CDR, Complementarity Determining Region) erreicht der Organismus über die V (D) J-Rekombination.

Die leichten Ketten bestehen aus jeweils einer variablen und einer konstanten Domäne. Bezeichnet werden diese als VL und CL. Die schweren Ketten hingegen haben jeweils eine variable und 3 konstante Domänen. Bezeichnet werden diese analog als VH und CH1, CH2, CH3.

Antikörper im Sinne der vorliegenden Erfindung sind ganze Antikörper oder Fab- oder scFv-Fragmente davon, vorausgesetzt, dass diese mindestens eingeschränkt in der Lage sind, an das Antigen zu binden.

Humanisierte und chimäre Antikörper sind ebenfalls durch die vorliegende Erfindung umfasst.

Ein solcher Antikörper und/oder das Antigen der vorliegenden Erfindung kann beispielsweise im Rahmen
- eines ELISA-Verfahrens zur Quantifizierung von Antigenen oder Antikörpern beispielsweise im Serum, in Zellkulturüberständen etc. mittels enzymgekoppelter Antikörper,
- eines ELISPOT-Verfahrens zum Nachweis von antikörper- oder antigensezernierenden Zellen (Plasmazellen) mittels enzymgekoppelter Antikörper,
- eines FACS-Verfahrens zur Quantifizierung von Zellen mittels fluoreszenzgekoppelter Antikörper gegen Antigene auf der Zelloberfläche, im Zytoplasma oder im Zellkern,
- eines Western Blots,
- eines Supergelshift-Experiments (auch EMSA),
- eines Phagen-Displays,
- eines Drugwipe-Tests,
- eines Abzyme-Verfahren oder
- eines Verfahrens zur Aufreinigung der Antigene bzw. Antikörper der vorliegenden Erfindung durch entsprechende Affinitätsverfahren
eingesetzt werden.

Wie das Antigen wird auch der Antikörper der vorliegenden Erfindung bevorzugt im Rahmen einer Zusammensetzung zur Verfügung gestellt, die wiederum - wie auch das Antigen - bevorzugt einen pharmazeutisch akzeptablen Träger umfasst.

Der Antikörper der vorliegenden Erfindung kann zur passiven Impfung gegen *Enterococcus faecalis* und/oder *Enterococcus faecium* verwendet werden und stellt so ein Vakzin gegen *Enterococcus faecalis* und/oder *Enterococcus faecium* dar.

Bei einer passiven Impfung wird mit Impfserum geimpft, welches den spezifischen Antikörper der vorliegenden Erfindung gegen *Enterococcus faecalis* und/oder *Enterococcus faecium* vorzugsweise in hoher Konzentration enthält.

Grundsätzlich sind die Zusammensetzungen der vorliegenden Erfindung bevorzugt pharmazeutische Zusammensetzungen. Diese zeichnen sich dadurch aus, dass sie für die Verabreichung im Rahmen einer Therapie oder Prophylaxe geeignet sind.

Ein weiterer Gegenstand der vorliegenden Erfindung ist ein Verfahren zum Detektieren von *Enterococcus faecalis-* und/oder *Enterococcus faecium*-Antikörpern, wobei das Verfahren die folgenden Schritte umfasst:
- das in-Kontakt-bringen von Antigenen gemäß der vorliegenden Erfindung mit einer auf *Enterococcus faecalis*- und/oder *Enterococcus faecium*-Antikörper zu testenden Probe und
- das Detektieren von Antikörper-Antigen- oder Antikörper-Antigenkonjugat-Komplexen,
wobei das Vorhandensein solcher Komplexe das Vorhandensein von *Enterococcus faecalis-* und/oder *Enterococcus faecium-*Antikörpern in der Probe anzeigt.

Ebenfalls durch die vorliegende Erfindung umfasst ist ein Verfahren zum Detektieren von *Enterococcus faecalis- und*/*oder Enterococcus faecium*-Antigenen mit mindestens den folgenden Schritten:
- in-Kontakt-bringen von Antikörpern gemäß der vorliegenden Erfindung optional immobilisiert auf einem Träger mit einer auf *Enterococcus faecalis- und*/*oder Enterococcus faecium*-Antigenen zu testenden Probe, und
- Detektieren von Antigen-Antikörper- oder Antikörperkonjugat-Antigen-Komplexen,
wobei das Vorhandensein solcher Komplexe das Vorhandensein von *Enterococcus faecalis- und*/*oder Enterococcus faecium*-Antigenen in der Probe anzeigt.

Es ist bei den beiden obigen Verfahren bevorzugt, dass die Antigene bzw. Antikörper auf einer Matrix, insbesondere auf einer festen Matrix, vorzugsweise auf einer Mikrotiterplatte, immobilisiert werden.

Alternativ ist es ebenso denkbar, dass die zu untersuchende Probe auf einer Matrix immobilisiert wird.

Beides erleichtert insbesondere die Detektion von gebildeten Antigen-Antikörperkonjugaten, da nicht gebundene Probenbestandteile optional von der Matrix, beispielsweise durch Waschen, entfernt werden können, bevor die Komplexe detektiert werden. Dies vermindert Rauschen und erhöht die Messgenauigkeit, ferner wird durch die Verwendung einer festen Matrix die maschinelle Handhabung der Proben erleichtert, so dass das Verfahren der vorliegenden Erfindung sich unter anderem ausgezeichnet für eine Automatisierung und somit beispielsweise für ein High-Throughput-Screening eignet.

Die Detektion der Antigen-Antikörperkonjugate kann weiter dadurch erleichtert werden, dass die Antigen, bzw. die Antikörper der vorliegenden Erfindung mit einem detektierbaren Marker versehen werden.

Ist beispielsweise die zu untersuchende potentiell anti-*Enterococcus faecalis- und*/*oder Enterococcus faecium-*Antikörper enthaltende Probe auf einer Matrix immobilisiert, und wird diese immobilisierte Probe dann mit markierten erfindungsgemäßen Antigenen in Kontakt gebracht, so können nach erfolgtem Waschen Proben, die anti-*Enterococcus faecalis- und*/*oder Enterococcus faecium*-Antikörper enthalten, leicht durch das Signal erkannt werden, das der Marker auf dem Antigen freisetzt.

Als Marker verwendbar ist jede Verbindung geeignet, die mit den Antikörpern, bzw. Antigenen, der vorliegenden Erfindung in Kontakt gebracht werden kann, ohne dass durch diesen Kontakt die Antigen-Antikörper-Wechselwirkungen vollständig unterbunden werden und ein wie auch immer geartetes detektierbares Signal direkt oder indirekt, gegebenenfalls nach entsprechender Aktivierung oder Substratvorlage, erzeugen.

Bevorzugte Marker im Sinne der vorliegenden Erfindung sind radioaktive Marker, farbige Marker, enzymatische Marker und magnetische Marker.

Das Detektionsverfahren der vorliegenden Erfindung kann weiter beschleunigt werden, wenn der Marker nach dem Binden an einen Antikörper oder an ein Antigen eine Eigenschaftsänderung zeigt. Hierdurch könnte man ungebundene markierte Antigene, bzw. Antikörper, von gebundenen unterscheiden, ohne dass beispielsweise ein Waschschritt erforderlich ist.

Die vorliegende Erfindung sieht ebenfalls ein Kit zum Detektieren von *Enterococcus faecalis* und/oder *Enterococcus faecium-Antikörpern* und/oder *Enterococcus faecalis* und/oder *Enterococcus faecium*-Antigenen vor, das das Antigen und/oder den Antikörper der vorliegenden Erfindung in einer der oben beschriebenen Ausführungsformen umfasst.

Insbesondere kann das erfindungsgemässe Antigen und/oder der erfindungsgemässe Antikörper an einen Träger, vorzugsweise an eine feste Matrix, gebunden sein und/oder mit einem Marker, vorzugsweise einem radioaktiven Marker, einem farbigen Marker, einem enzymatischen Marker oder einem magnetischen Marker markiert sein.

Weiterhin umfasst die vorliegende Erfindung die Verwendung des erfindungsgemässen Antigens gemäss Anspruch 1 bis 7 zur Detektion oder Herstellung von *Enterococcus faecalis* und/oder Enterococcus faecium-Antikörpern und/oder Fab- oder scFv-Antikörperfragmenten.

Die so hergestellten *Enterococcus faecalis* und/oder Enterococcus faecium-Antikörper und/oder Fab- oder scFv-Antikörperfragmente sind insbesondere zur passiven Immuntherapie oder zur Prophylaxe gegen Enterokokkenantigene vorgesehen.

Herstellbar sind die erfindungsgemässen Antikörper oder Fab- oder scFv-Antikörperfragmente, beispielsweise mit einem Verfahren, umfassend die Verabreichung des erfindungsgemässen Antigens an ein Tier, insbesondere ein Säugetier in einer Menge, die ausreicht, um Antikörper oder Antikörperfragmente herzustellen.

Zur Herstellung von polyklonalen Antikörpern kann zunächst das erfindungsgemässe Antigen, gegen das der Antikörper gerichtet sein soll, ausgewählt und produziert werden. Dies kann auf verschiedene Weisen erreicht werden, zum Beispiel, indem ein Antigen aus *Enterococcus faecalis* und/oder *Enterococcus faecium* isoliert wird, in vitro synthetisiert wird oder rekombinant, beispielsweise in Bakterien, hergestellt wird. Anschliessend wird das Antigen einem Tier verabreicht, dessen Immunsystem dann Antikörper gegen das Antigen bildet. Als Antikörper-Produzenten kommen insbesondere Mäuse, Ratten und Kaninchen, aber auch Ziegen, Schafe und Pferde in Betracht. Bevorzugt wird die Immunisierung mehrfach wiederholt. Nach ein paar Wochen werden dem Blut bzw. dem Serum des Antikörperproduzenten polyklonale Antikörper entnommen.

Zur Herstellung von monoklonalen Antikörpern können - wie bei der polyklonalen Antikörperherstellung beschrieben - Tiere, bevorzugt Mäuse und/oder Ratten immunisiert und dann deren Plasmazellen (aus Milz oder Lymphknoten) gewonnen. Diese Plasmazellen können mit Tumorzell-Linien verschmolzen werden, und so können sog. Hybridoma-Zell-Linien erzeugt werden, die theoretisch unendlich lange leben, aber eine einzige Art von monoklonalen Antikörpern sezernieren, die wiederum aus dem Zellkulturüberstand isoliert werden können.

Eine weitere Ausführungsform der vorliegenden Erfindung ist ein Verfahren zur Herstellung eines erfindungsgemässen Antikörpers oder eines Fab oder scFv-Fragmentes davon, in rekombinanter Form, umfassend das Klonieren einer DNA-Sequenz, die für diesen Antikörper oder dessen Fragment kodiert, in einen Expressionsvektor, das Transformieren einer Zelle, insbesondere einer E. coli - Zelle oder einer Hefezelle oder einer eukaryoten Zelle (wie z.B. einer CHO-Zelle) mit diesem Konstrukt und die Expression des rekombinanten Antikörpers oder eines Fragmentes davon.

Ein durch ein solches Verfahren erhältlicher rekombinanter Antikörper ist dadurch gekennzeichnet, dass er strukturell den Antikörpern der vorliegenden Erfindung entspricht. Ebenso entsprechen rekombinante Fab oder scFv-Fragmente des erfindungsgemässen Antikörpers, den nativ aufreinigbaren Antikörperfragmenten der vorliegenden Erfindung.

Die rekombinanten Antikörper und Antikörperfragmente der vorliegenden Erfindung haben insbesondere den Vorteil, dass sie ohne grossen technischen Aufwand in grosser Menge herstellbar sind, ohne dass Tiere als Antikörperproduzenten dienen müssen. Es ist ferner eine erheblich grössere Reinheit der Antikörperpräparationen erzielbar, und der direkte Kontakt mit Blut und die damit verbundene Infektionsgefahr wird vermieden.

Es ist dem Fachmann klar, dass er sämtliche Ausführungsformen der vorliegenden Erfindung, die hierin beispielhaft aufgeführt sind, beliebig miteinander kombinieren kann, ohne hierbei vom Umfang der Offenbarung dieser Erfindung abzuweichen.

Weitere Vorteile und Ausführungsformen der Erfindung ergeben sich aus der Beschreibung einzelner Ausführungsbeispiele sowie aus der Zeichnung.

Es zeigt:
Figur 1: Das ¹H NMR-Spektrum des kapsulären Polysaccharids aus *E. faecalis* Stamm Typ 5. Das Spektrum wurde bei 600 MHz und 27°C aufgenommen. Die Buchstaben beziehen sich auf die Kohlenhydrat-Reste, wie in Fig. 3 gezeigt, und die arabischen Zahlen beziehen sich auf die Protonen an den entsprechenden Resten; LA, Milchsäure.
Figur 2: Ausschnitte aus dem ROESY-Spektrum von dem kapsulären Polysaccharid aus *E. faecalis* Stamm Typ 5. Das Spektrum wurde bei 600 MHz und bei 27°C aufgenommen. Die Buchstaben beziehen sich auf die Kohlenhydrat-Reste, wie in Fig. 3 gezeigt, und die arabischen Zahlen beziehen sich auf die Protonen an den entsprechenden Resten. Die NOE-Kontakte zwischen den Resten sind unterstrichen und in Schrägschrift dargestellt.
Figur 3: Die chemische Struktur der sich wiederholenden Einheit des kapsulären Polysaccharids aus *E. faecalis* Stamm Typ 5. LA, Milchsäure. Die D-Konfiguration des Gal***f*** Restes wird vermutet.
Figur 4: Binden eines Kaninchen-Antiserums gegen die bakteriellen Zellen von *E. faecalis* Stamm Typ 5. Die verwendeten Antigene sind in der Legende angegeben. Die angegebenen Werte stellen jeweils den Durchschnitt aus mindestens zwei Messungen dar.
Figur 5: Inhibierung der Opsonophagozytose von Bakterienzellen vom *E. faecalis* Stamm Typ 5 durch Kaninchen-Antiserum. Alle Antiseren wurden in einer 1:200 Verdünnung verwendet. Die Inhibitoren sind in der Legende angegeben. Die angegebenen Daten stellen einen Durchschnitt von mindestens 4 Messwerten dar, und die Fehlerbalken repräsentieren den SEM. Die Opsonophagozytose-Aktivität ohne Inhibitor betrug > 70% für alle Antiseren.
Figur 6: Bindung von in Kaninchen erzeugten Antikörpern gegen kapsuläres Polysaccharid aus *E. faecalis* Typ 2 und Typ 5 gegen das entsprechende gereinigte Antigen.
Figur 7: Bestimmung der Abtötung durch Opsonophagozytose von *E. faecalis* Typ 2, die gegen das gereinigte Antiserum erzeugt wurden. Die Werte belegen eindeutig, dass in Kaninchen schützende Antikörper erzeugt werden konnten, die im *in vitro-*Test zur Abtötung der Bakterien führten.

### Beispiel 1

Die Eliminierung von *Enterococcus faecalis* durch Opsonophagozytose wird unter anderem durch Antikörper bewerkstelligt, die gegen Kohlenhydrat-Antigene der Zellwand und der bakteriellen Kapsel gerichtet sind. Für den *E*. *faecalis* Stamm 12030, wurde Lipoteichonsäure (LTA) kürzlich als Ziel der opsonisierenden Antikörper identifiziert. Jedoch tötet Serum, das gegen aufgereinigtes LTA gezogen wurde, keine bakteriellen Stämme mit dem CPS-C und -D-Serotyp.

Im Rahmen dieses Beispiels wird ein neues kapsuläres Polysaccharid aus dem *E. faecalis* Typ 5-Stamm, einem CPS-D-Stamm, durch enzymatischen Verdau der Zellwand sowie durch Gel-Permeation und durch Anionen-Austausch-Chromatographie isoliert.

Das isolierte Polysaccharid wird durch Zuckeranalyse, eindimensionale und zweidimensionale homonukleare and heteronukleare ¹H und ¹³C NMR Spektroskopie analysiert.

Es wird ein neues kapsuläres Polysaccharid aus *E. faecalis* identifiziert, das eine ungewöhnliche →6)-3-O-[1-carboxyethyl]-β-Gal***f***-(1→ - Einheit in der sich wiederholenden Einheit enthält.

Kaninchenantiserum, das mittels Immunisierung von hitzeinaktivierten Bakterienzellen vom *E. faecalis* Typ 5 induziert wurde, enthielt sowohl Antikörper spezifisch gegen das neue kapsuläre Polysaccharid wie gegen LTA dieses Stammes.

Die Opsonophagozytose von *E. faecalis* type 5 durch dieses Antiserum wurde jedoch nur durch das aufgereinigte Polysaccharid, aber nicht durch LTA, inhibiert.

Somit illustriert dieses Beispiel eine Möglichkeit, wie ein neues kapsuläres Polysaccharid als Antigen in *E. faecalis* type 5 identifiziert werden kann, das immunogen ist und das als Target für opsonisierende Antikörper dienen kann.

Dieses Beispiel illustriert ferner, wie die Immunogenität von Antigen, die von *E. faecalis* abgeleitet sind, getestet werden kann.

### Beispiel 2:

### 2a) Bakterienstämme und Kulturen

Kapsuläre Polysaccharide wurden aus *E. faecalis* Typ 5 (Maekawa, S., et al., Microbiol. Immunol., 36:671-681, 1992), einem CPS-D Stamm, mit einem neulich beschriebenen Serotypisierungssystem (Hufnagel, M., et al., J. Clin. Microbiol. 42:2548-2557, 2004) isoliert. Bakterienzellen wurden aus den Ausgangskulturen in einer Columbia-Nährlösung (Becton Dickinson, Sparks, MD, USA), die mit 1% Glucose angereichert war, ohne Agitation bei 37°C 2 Stunden lang kultiviert.

### 2b) Antiseren

In der Vergangenheit wurden bereits Antiseren gegen ganze Bakterienzellen von *E. faecalis* Typ 5 beschrieben (Hufnagel, M., et al., J. Clin. Microbiol. 42:2548-2557, 2004). Das Antiserum gegen LTA wurde mit LTA, das aus dem Stamm 12030 wie vorher beschreiben (Theilacker, C., et al., Infect. Immun. 74, 2006) gereinigt wurde, hergestellt. Ein weibliches weißes Neuseeland-Kaninchen wurde subkutan mit 100 µg LTA, das in vollständigem Freund-Adjuvans suspendiert war, immunisiert und danach mit derselben Dosis LTA, das in unvollständigem Freund-Adjuvans suspendiert war, sieben Tage später und danach in der folgenden Woche alle drei Tage mit Nachimpfungsdosen von 10 µg immunisiert.

### 2c) Herstellung und Charakterisierung des kapsulären Polysaccharids.

LTA aus *E. faecalis* wurde wie vorher beschrieben (Huebner, J., et al., Infect. Immun. 67:1213-1219, 1999; Theilacker, C., et al., Infect. Immun. 74, 2006, isoliert. Das hier beschriebene Antigen wurde folgendermaßen präpariert: Kurz zusammengefasst wurden Bakterienzellen durch Zentrifugieren geerntet und durch das Hinzufügen von Mutanolysin und Lysozym (jeweils 100 pg/ml, Sigma Chemicals, St. Louis, MO, USA in PBS angereichert mit 5 mM MgCl₂, 1 mM CaCl₂ und 0.05% NaN₃) bei 37°C 18 Stunden lang verdaut. Unlösliches Material wurde abzentrifugiert, und der Überstand wurde mit Nukleasen (DNase I und RNase A, 100 pg/ml) bei 37°C 4 Stunden lang behandelt, gefolgt von einer 18-stündigen Hinzufügung von Proteinase K (100 pg/ml, alle erhältlich von Sigma Chemicals) bei 56°C. Der Überstand wurde durch das Hinzufügen von Ethanol (Endvolumen 80%) präzipitiert und dann durch Zentrifugieren gesammelt. Nach der Dialyse gegen entionisiertes H₂0, wurde das Material lyophilisiert. Für eine Gelpermeationschromatographie wurde das Material in 0,01 M Ammoniumhydrogencarbonatpufferlösung aufgelöst und auf eine Sephacryl S-400-Säule (1,6 x 90 cm) (GE Healthcare, Uppsala, Schweden) aufgetragen. Fraktionen, die bei einem Kₐᵥ von etwa 0,45 eluierten, wurden kombiniert, dialysiert und lyophilisiert. Das Material wurde in 20 mM NaHC0₃, pH 8,4 resuspendiert und auf eine Anionenaustauschsäule (Sepharose Q FF, GE Healthcare) aufgetragen. Gebundenes Antigen wurde durch einen linearen NaCl-Gradienten aus der Säule eluiert und Fraktionen, die Polysaccharide umfassten, wurden durch einen Dubois-Assay (Dubios, M., et al., Anal. Chem. 28:350-356, 1956.) und Immunoblotten unter Verwendung eines Kaninchen-Anti-Typ 5 Immunserums identifiziert. Immunoreaktives Material, das bei 450 mM NaCl eluiert, wurde kombiniert, dialysiert und lyphilisiert. Als abschließenden Reinigungsschritt wurde eine Gelpermeationschromatographie in einer 1,5 x 75 cm Toyopearl HW-40 (Tosoh Corporation, Tokyo, Japan)-Säule durchgeführt. Die Reinheit des isolierten Materials wurde mittels einer SDS-PAGE unter Verwendung eines 10% Bis-Tris-Gels und eines MOPS-Laufpuffers (Invitrogen, Karlsruhe, Germany) und durch Coomassie (Invitrogen) und PAS (Sigma)-Färbung gemäß der Anleitung des Herstellers bestätigt.

Ferner wurde ein Western-Blot von Material, das durch SDS-PAGE abgetrennt wurde, mit einem Anti-Typ 5 Kaninchenantiserum gefärbt.

### 2d) Ergebnis der Reinigung des kapsulären Polysaccharids

Kapsuläres Polysaccharid aus dem E. faecalis Stamm Typ 5 wurde durch enzymatischen Verdau von Peptidoglycan aus den Bakterienzellen mobilisiert. Das extrahierte Material eluierte als zwei Kohlenhydrat-haltige Fraktionen bei der Sephacryl S-400 Gelchromatographie. Eine Fraktion, die im Ausschlussvolumen eluierte, bestand aus LTA, wie durch ¹H NMR-Analyse (Daten nicht angegeben) bestimmt. Eine große, zweite Fraktion bei einem Kₐᵥ im Bereich von 0,45 wurde durch eine Anionenaustauschchromatographie unter Verwendung von Q-Sepharose weiter gereinigt. Geringe Mengen an immunoreaktivem Material eluierten bei 450 mM NaCl, das nur aus Glucose und Galactose bestand, und das nach der Gelpermeationschromatographie auf Toyopearl HW-40S einer weiteren Analyse unterzogen wurde. Die SDS-PAGE mit diesem gereinigten Material zeigte eine einzelne breite Bande bei 100 kDa, das sich mit PAS färbte, aber nicht bei Commassie-Blau. Ein Western-Blot, das mit Anti-Typ 5 Antiserum gefärbt wurde, zeigte ebenfalls eine einzelne breite Bande bei 100 kDa und keine weiteren Banden (Daten nicht angegeben).

### Beispiel 3

### Herstellung von LTA

LTA wurde durch Butanolextraktion und hydrophobe Interaktionschromatographie wie vorher beschrieben (Theilacker, C., et al., Infect. Immun. 74, 2006) hergestellt. Die Reinheit der LTA-Präparate wurde durch SDS-PAGE und Western-Blot-Analyse mit dem entsprechenden Antiserum gegen ganzen Bakterienzellen (vgl. oben) bewertet. Die strukturelle Identität von LTA wurde durch NMR-Spektroskopie wie kürzlich beschreiben (Theilacker, C., et al., Infect. Immun. 74, 2006) bestätigt.

### Allgemeine und Analytische Verfahren

Die Hydrolyse wurde mit 2 M Trifluoressigsäure (120°C, 3 h) durchgeführt. Monosaccharide wurde zu Alditolacetaten umgewandelt und durch GC auf einem Hewlett-Packard 5890 Chromatograph mit einer SPB-5-Säule (30 m x 0,25 mm x 0,25 pm, Supelco, München, Deutschland) unter Verwendung eines Temperaturprogrammes 150°C für 3 Minuten, dann 3°C min⁻¹ bis zu 300°C analysiert. Die absolute Anordnung der Zuckerreste wurde wie vorher beschrieben (Haseley, S. R., et al., Eur. J. Biochem. 244:761-766, 1997; Leontein, K., et al., Carb. Res. 62:359-362,1978) bestimmt.

### NMR-Spektroskopie

Die Probe wurde drei Mal mit 99,0% ²H₂0 ausgetauscht, lyophilisiert, und in 99,9% ²H₂0 redispergiert. Alle ein- und zweidimensionalen Spektren wurden mit einem Bruker DRX Avance 600 MHz Spektrometer (Arbeitsfrequenzen 600,31 MHz bei ¹H NMR und 150,96 MHz bei ¹³C NMR) unter Verwendung einer gewöhnlichen Bruker Software (Bruker, Rheinstetten, Germany) bei 27°C aufgezeichnet. Die chemischen Verschiebungen sind relativ zu Aceton (ÖH 2,225; δC 31,45) angegeben. Korrelationsspektroskopie (COSY), Gesamtkorrelationsspektroskopie (TOCSY), und ROESY wurden unter Verwendung von Datenblättern (t1 x t2) mit 4096 x 512 Punkten aufgezeichnet, und 32 Scans wurden durchgeführt.

TOCSY und ROESY wurden auf eine Phasen-sensitive Art und Weise gemäß dem Verfahren von States et al. durchgeführt und für TOCSY wurde eine Mischzeit von 100 ms verwendet (States, D. J., et al., J. Magn. Reson. 48:286-292, 1982). Die ¹H, ¹³C-Korrelationen wurden in dem ¹H-Detektionsmodus mittels einer Multiplen-Quantum-Koherenz (HMQC) mit Protonenentkoppelung in der ¹³C-Domäne unter Verwendung von Datensets mit 2048 x 256 Punkten gemessen, und für jeden t₁ -Wert wurden 128 Scans aufgenommen (Bax, A., et al., J. Am. Chem. Soc. 109:2093-2094, 1986; Summers, M. F., et al., J. Am. Chem. Soc. 108:4285-4294, 1986).

### Chemische Analyse und NMR-Spektroskopie

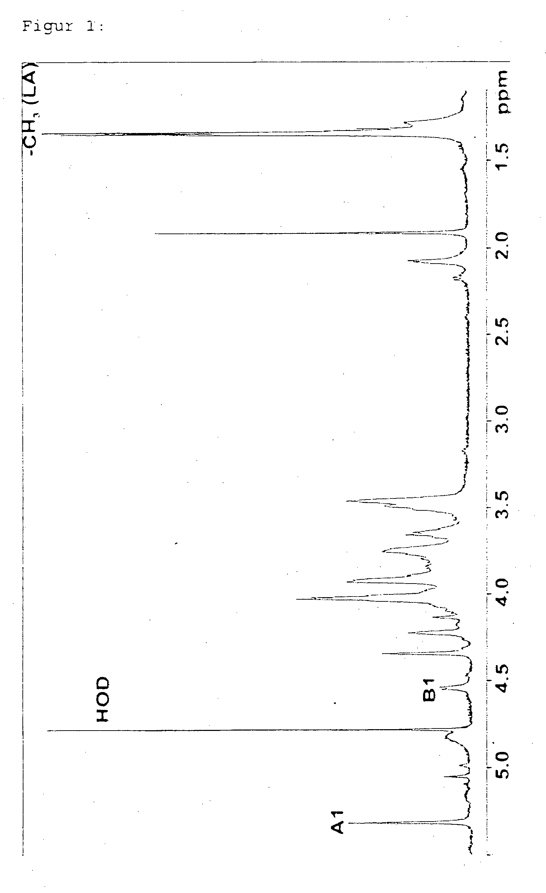

Im niedrig-Feld-Bereich zeigte das ¹H NMR-Spektrum des aufgereinigten Polysaccharids (Fig. 1) zwei anomere Signale bei δ 5.315 (Rest A, [³J_{H1,H2} <2 Hz]), und bei δ 4.542 (Rest B, [³J_{H1,H2} = 7.8 Hz]), welche als β-Gal*f* und β-D-Glcp identifiziert wurden. Weiterhin wurde das Dublett bei δ 1.361 als eine Methylgruppe erkannt, die zu einem Milchsäurerest (LA) gehört (Knirel, Y. A., et al., Carbohydr. Res. 259, 1994; , Knirel, Y. A., et al., Carbohydr. Res. 235:C19-23, 1992). Die Gegenwart eines D-Glc***p***-Restes wurde durch chemische Analyse, der Zuweisung der absoluten Konfiguration, sowie durch NMR-Spektroskopiedaten bestätigt. Der mit Milchsäure an Position C-3 substituierte Gal*f*-Rest wurde nur durch NMR-Daten identifiziert (Beynon, L. M., et al., Eur. J. Biochem. 250:163-167, 1997; Knirel, Y. A., et al., Carbohydr. Res. 259, 1994; , Knirel, Y. A., et al., Carbohydr. Res. 235:C19-23, 1992). Alle ¹H und ¹³C chemischen Verschiebungen des kapsulären Polysaccharids aus *E. faecalis* Typ 5 (Tab. 1) wurden aus ¹H,¹H COSY and TOCSY, und aus ¹H,¹³C HMQC Spektren bestimmt.

**-Tabelle 1-**

| Rest | Chemische Verschlebung ¹H and ¹³C [δ] | | | | | | |
|---|---|---|---|---|---|---|---|
| | H1 | H2 | H3 | H4 | H5 | H6^{a} | H6^{b} |
| | C1 | C2 | C3 | C4 | C5 | C6 | |
| A | 5.315 | 4.346 | 3.932 | 4.225 | 4.040 | 3.768 | 4.019 |
| →6)-β-Gal*f*- | 109.26 | 80.26 | 84.96 | 82.41 | 70.55 | 71,90 | |
| B | 4.542 | 3.460 | 3.663 | 3.465 | 3.500 | 3,742 | 3.930 |
| →3)-β-D-Glc*p*- | 103.22 | 74.05 | 82.44 | 68,80 | 76.26 | 61.26 | |
| LA | | 4.038 | 1.361 | | | | |
| Milchsäure | 181.29 | 77.72 | 19.21 | | | | |

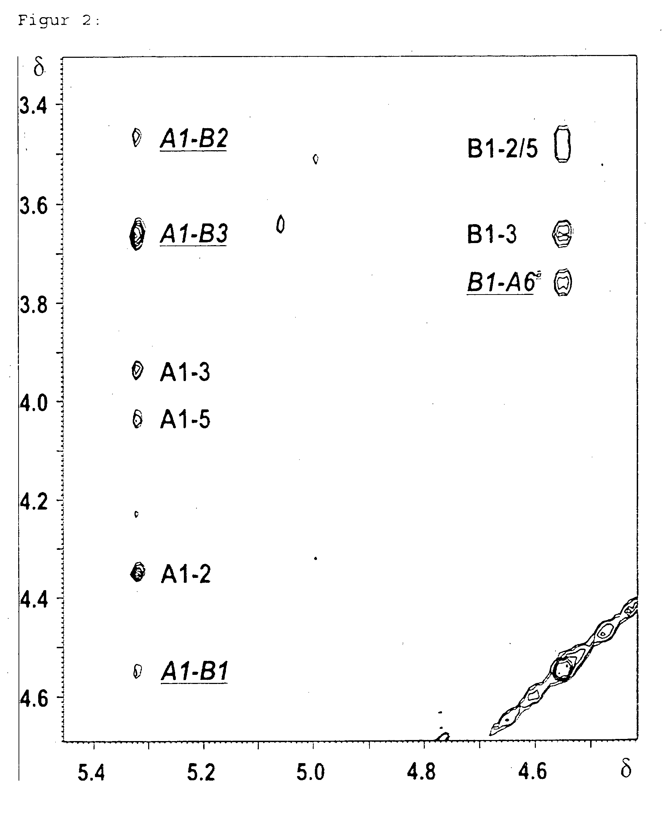

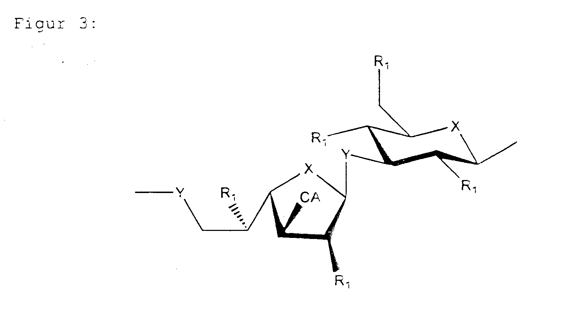

Ins Niedrigfeld verschobene Signale der Kohlenstoffatome zeigten Substitutionen an C-6 und C-3 von β-Galf (Rest A, δ 71.90 und δ 84.96) und Substitutionen an C-3 von β-D-Glcp (Rest B, δ 82.44) an. Die Sequenz der Reste in der sich wiederholenden Einheit wurde durch ROESY Experimente bestimmt. Starke NOE-Kontakte zwischen den Resten wurden zwischen den Protonen A1 (δ 5.315) und B3 (δ 3.663), und B1(δ 4.542) und A6a (δ 3.768) (Fig. 2) gefunden. Somit war die Struktur der sich wiederholenden Einheit des isolierten Polysaccharids so, wie sie in Figur 3 dargestellt ist.

### Beispiel 4

### ELISA Untersuchungen

ELISA-Experimente wurden durch herkömmliche Verfahren wie vorher beschrieben (Theilacker, C., et al., Infect. Immun. 74, 2006) durchgeführt. Kurz gesagt wurden Mikrotiterplatten mit verschiedenen Kohlenhydratantigenen, die von *E*. *faecalis* (10 pg/ml in 0,04 M Phosphatpuffer, pH 7,0) abstammen, beschichtet, und 18 Stunden lang bei 4°C belassen. Mit PBS, das 0,05% Tween 20 enthielt, wurden Waschschritte durchgeführt. Die Platten wurden mit 3% Magermilch in PBS-0,02% Natriumazid 2 Stunden lang bei 37°C blockiert. Ein Ziegen Anti-Kaninchen IgG Alkaliphosphatasekonjugat (Sigma), verdünnt auf 1:1000, wurde als sekundärer Antikörper verwendet, und p-Nitrophenylphosphat wurde als Substrat verwendet (Sigma). Nach 60-minütiger Inkubation bei 37°C wurde die Absorption bei 405 nm gemessen.

### Beispiel 5

### Opsonophagozytose-Assay

Ein Opsonophagozytose-Assay wurde wie vorher beschreiben (Theilacker, C., et al., Infect. Immun. 74, 2006) ausgeführt. Babykaninchenserum (Cedarlane Laboratories, Hornby, Ontario, Canada), das mit dem Targetbakterienstamm absorbiert wurde, diente als Komplementquelle. Die opsonische Aktivität der Immunseren wurde mit derjenigen der Kontrollen, die normales Kaninchenserum enthielten, verglichen. Das Immunserum wurde vor der Verwendung 30 Minuten lang bei 56°C durch Hitze inaktiviert. Negative Kontrollen umfassten Probenröhrchen, die entweder keine polymorphonuklearen Leukozyten, kein Komplement oder kein Serum enthielten. Die opsonische Aktivität des Serums wurde wie folgt berechnet: [1 - (CFU Immunserum bei 90 min/CFU Preimmunserum bei 90 min)] x 100.
Zur Untersuchung der Inhibierung Opsonophagozytose wurde Antiserum in einer Konzentration von 1:200 60 Minuten lang bei 4°C mit 0,08 bis 100 µg/ml inkubiert. Nach der Inkubation wurde das absorbierte Serum hinzugefügt und der Opsonophagozytose-Assay wurde wie oben beschrieben fortgesetzt. Ohne Inhibierung wiesen alle Seren eine minimale Opsonophagozytose-Aktivität von > 70% des Inokulums auf.

### Beispiel 6

### Immunochemische Charakterisierung

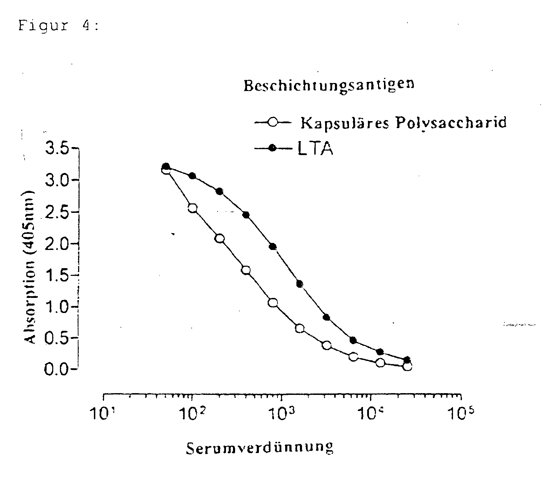

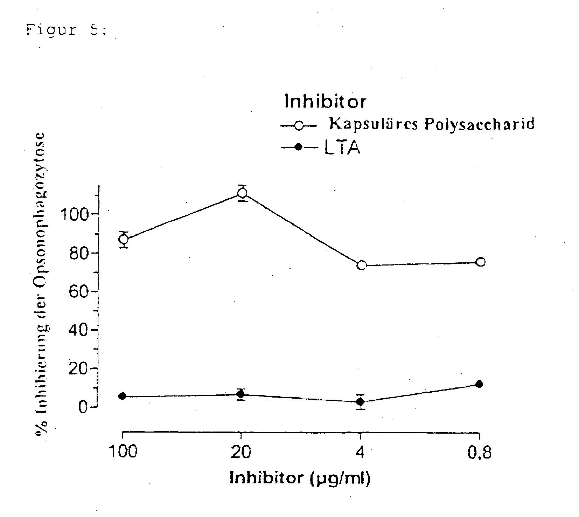

Serum gegen Bakterienzellen von *E*. *faecalis* type 5 (Maekawa, S., et al., Microbiol. Immunol. 36:671-681, 1992) war gegenüber dem aufgereinigtem Polysaccharid reaktiv (Fig. 4). Das Antiserum enthielt ebenso hohe IgG Antikörperspiegel gegen *E. faecalis* LTA (Fig. 4). Da anti-LTA-Antikörper die Opsonophagozytose des *E. faecalis*-Stammes 12030 vermitteln, ist angestrebt, die Spezifität opsonisierender Antikörper gegenüber dem Stamm *E*. *faecalis* Typ 5 zu ermitteln. In Übereinstimmung mit der Beobachtung, dass es wenig Kreuz-Reaktivität von opsonisierenden Antikörpern zwischen *E*. *faecalis* Typ 5 und *E*. *faecalis* 12030 gibt (Hufnagel, M., et al., J. Clin. Microbiol. 42:2548-2557, 2004), inhibierte Aufgereinigtes LTA die Opsonophagozytose-Aktivität von anti-Typ 5-Serum nicht (Fig. 5). Jedoch war das aufgereinigte kapsuläre Polysaccharid ein potenter Inhibitor opsonisierender Antikörper gegen Typ 5 (Fig. 5). Kaninchenserum gegen aufgereinigtes LTA, das die Opsonophagozytose des Stammes 12030 in dem Opsonophagozytose-Assay fördert, war gegen den Stamm Typ 5 nicht opsonierend, was die Ergebnisse aus unseren Inhibierungsstudien bestätigt (Daten nicht gezeigt).

### Beispiel 7

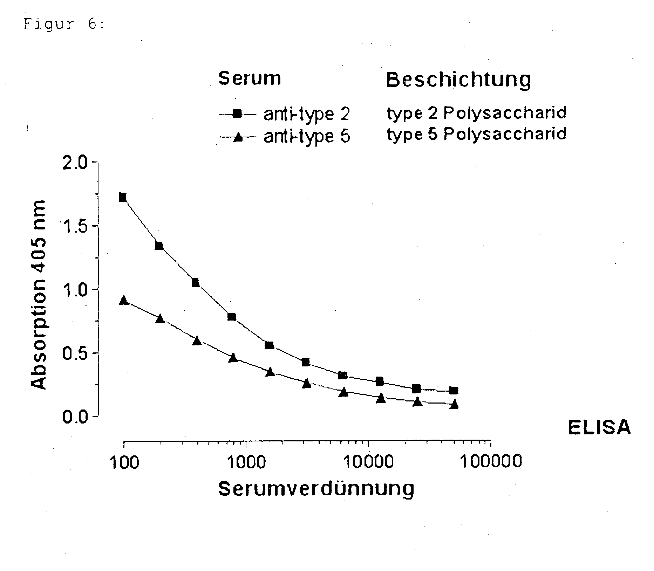

Gegen die beiden Polysaccharide vom Typ 2 sowie vom Typ 5 wurden Antikörper in Kaninchen erzeugt: Ein weibliches weißes Neuseeland-Kaninchen wurde subkutan mit 100 µg gereinigtem Polysaccharid vom Typ 2 und Typ 5, das in vollständigem Freund-Adjuvans suspendiert war, immunisiert und danach mit derselben Dosis Polysaccharid, das in unvollständigem Freund-Adjuvans suspendiert war, sieben Tage später und danach in der folgenden Woche alle drei Tage mit Nachimpfungsdosen von 10 µg immunisiert.
Die Polysaccharide wurden nach der unter 2d) beschriebenen Methode gewonnen. Für die Immunisierung wurden jeweils zwei Kaninchen eingesetzt und anschließend das Antiserum von den Kaninchen gewonnen. Die von den Kaninchen erhaltenen Antiseren wurden in einem ELISA-Test untersucht. Hierzu wurde sowohl das Polysaccharid vom Typ 2, wie auch das Polysaccharid vom Typ 5 auf die Mikrotiterplatten gebunden. Überraschenderweise wurde festgestellt, dass sowohl gegen gereinigtes Polysaccharid vom Typ 5, wie auch gegen Polysaccharid vom Typ 2 Antikörper gebildet wurden. Damit konnte gezeigt werden, dass das Antigen immunogen ist, was in Anbetracht der Tatsache, dass es sich um ein sogenanntes T-Zell-unabhängiges Antigen handelt, nicht unbedingt zu erwarten war. Die Bildung der Antikörper ist in Figur 6 dargestellt.

### Beispiel 8

### Opsonophagozytischer Test

Bei diesem in-vitro-Test wurden Granuluzyten, Antiserum sowie Bakterien zusammengebracht und es wurde überprüft, ob durch die erzeugten Antikörper eine Abtötung der *Enterococcus faecalis*-Bakterien erreicht werden konnte. Die genauen Bedingungen des Testabsatzes waren wie folgt: frisches Vollblut wurde von gesunden Spendern gewonnen und mit einem Heparin-Dextran-Puffer versetzt. Anschliessend wurden die weissen Blutzellen gereinigt und auf eine definierte Anzahl (5 X 10⁶ Zellen/ml) eingestellt. Die zu untersuchenden Bakterien wurden aus einer Kultur in mittlerem logarithischen Wachstum abzentrifugiert und spektrophotometrisch ebenfalls auf 5 x 10⁶ Zellen/ml eingestellt. Als Komplementquelle wurde lyophilisiertes Baby-Kaninchenserum verwendet, das 1:15 mit Zellkulturmedium verdünnt und mit dem Ziel-Bakterienstamm absorbiert wurde, um vorhandene Antikörper gegen den verwendeten Enterokokkenstamm zu entfernen. Das Kaninchenserum wurde ebenfalls mit Zellkulturmedium entsprechend des Versuchsaufbau verdünnt. Für den Versuch wurden jeweils 100 µl der Bakteriensuspension, 100 µl der Leukozyten (Verhältnis Bakterien zu Leukozyten 1:1), 100 µl der Komplementquelle sowie 100 µl der entsprechenden Antikörperverdünnung gemischt. Die Ausgangs-Keimzahl wurde durch Verdünnung und Ausplattierung festgestellt und der Versuchsansatz anschliessend bei 37 C für 90 Minuten in einem Überkopf-Rotor inkubiert. Zum Versuchsende wurden die Bakterien im Versuchsansatz ebenfalls wieder verdünnt und ausplattiert und die Anzahl der Kolonien am nächsten Tag gezählt. Die Reduktion der Bakterienzahl zwischen Inokulum und Keimzahl bei Versuchsende wurde als opsonophagozytose-vermittelte Abtötung in Prozent ausgedrückt. Dieser Wert stellt den besten Surrogatmarker für eine protektive Immunantwort gegen bakterielle Infektionserreger dar.

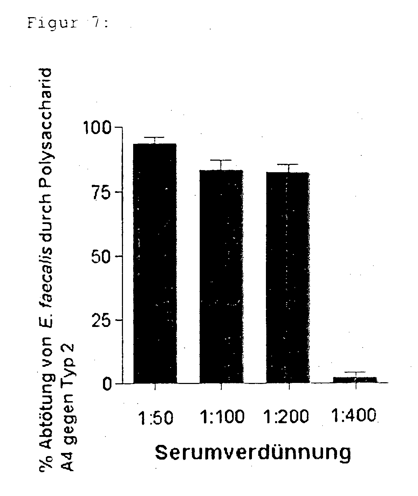

Die Ergebnisse des Versuches sind in Figur 7 dargestellt. Es zeigt sich, dass Serum-Verdünnungen von 1:50 bis 1:200 zu einer deutlichen Abtötung von ca. 80% führen, während bei weiteren Verdünnungen des Serums dieser Effekt verloren geht. Dies ist als Hinweis darauf zu verstehen, dass höhere Antikörpertiter gegenüber diesem Antigen einen Schutz des Organismus vor der Infektion mit E. faecalis darstellen.

## Patentansprüche

1. *Enterococcus faecalis* und/oder *Enterococcus faecium*-Antigen*,* **dadurch gekennzeichnet, dass** es eine aus einer Furanose Gal und aus einer Pyranose Glc bestehende Disaccharidstruktur aufweist, wobei die Disaccharidstruktur die folgende Formel aufweist: 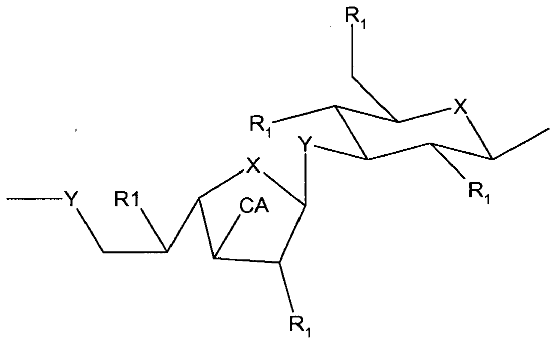
wobei R₁ unabhängig voneinander OH, OAc, OCₙHₘ, OFo, wobei Fo Formyl,
m = 2n + 1 und n eine natürliche Zahl von 1 bis 10 ist,
X = O,
Y = O und
CA unabhängig voneinander ein C₁-C₆-Acyl- oder ein Hydroxyacylrest ist.

2. Antigen gemäss Anspruch 1, **dadurch gekennzeichnet, dass** R₁ = OH, X = O, Y = O und CA = Lactyl ist.

3. Antigen nach Anspruch 1 oder 2, **dadurch gekennzeichnet, dass** es ein Molekulargewicht von ca. 1000-200000 Da, mehr bevorzugt von ca. 50000-150000 Da, besonders bevorzugt von ca. 100000 Da aufweist.

4. Antigen nach einem der Ansprüche 1 bis 3, **dadurch gekennzeichnet, dass** die Einheit pro Antigen mindestens 5 mal, bevozugt mindestens 10 mal, mehr bevorzugt mindestens 100 mal, besonders bevorzugt mindestens 1000 mal vorkommt.

5. Antigen nach einem der Ansprüche 1 bis 4, **dadurch gekennzeichnet, dass** es an einen Träger gebunden ist.

6. Antigen nach Anspruch 5, **dadurch gekennzeichnet, dass** die Bindung an den Träger kovalenter Natur ist.

7. Antigen nach Anspruch 5 oder 6, **dadurch gekennzeichnet, dass** der Träger ein Immunträger ist.

8. Monoklonaler Antikörper spezifisch gegen ein Antigen gemäss einem der Ansprüche 1 bis 7.

9. Zusammensetzung, umfassend ein Antigen gemäss einem der Ansprüche 1 bis 7 oder einen Antikörper gemäss Anspruch 8.

10. Zusammensetzung nach Anspruch 9, umfassend einen pharmazeutisch akzeptablen Träger.

11. Zusammensetzung nach Anspruch 9 oder 10, **dadurch gekennzeichnet, dass** sie ein Vakzin gegen *Enterococcus faecalis* und/oder *Enterococcus faecium* ist.

12. Zusammensetzung nach einem der Ansprüche 9 bis 11, **dadurch gekennzeichnet, dass** sie eine pharmazeutische Zusammensetzung ist.

13. Verfahren zum Detektieren von *Enterococcus faecalis* und/oder *Enterococcus faecium*-Antikörpern umfassend
- in-Kontakt-bringen von Antigenen gemäss Anspruch 1-7 mit einer *auf Enterococcus faecalis-* und/oder *Enterococcus faecium*-Antikörper zu testenden Probe, und
- Detektieren von Antikörper-Antigen- oder Antikörper-Antigenkonjugat-Komplexen, wobei das Vorhandensein solcher Komplexe das Vorhandensein von *Enterococcus faecalis-* und/oder *Enterococcus faecium*-Antikörpern in der Probe anzeigt.

14. Verfahren zum Detektieren von *Enterococcus faecalis* und/oder *Enterococcus faecium*-Antigenen, umfassend
- in-Kontakt-bringen von optional auf einem Träger immobilisierten monoklonalen Antikörpern gemäss Anspruch 8 mit einer auf *Enterococcus faecalis-* und/oder *Enterococcus faecium*-Antigenen zu testenden Probe, und
- Detektieren von Antigen-Antikörper- oder Antikörperkonjugat-Antigen-Komplexen, wobei das Vorhandensein solcher Komplexe das Vorhandensein von *Enterococcus faecalis-* und/oder *Enterococcus faecium*-Antigenen in der Probe anzeigt.

15. Verfahren nach Anspruch 13 oder 14, **dadurch gekennzeichnet, dass** die Antigene bzw. Antikörper auf einer Matrix, insbesondere auf einer festen Matrix, vorzugsweise auf einer Mikrotiterplatte, immobilisiert werden.

16. Verfahren nach Anspruch 13 bis 15, **dadurch gekennzeichnet, dass** das Antigen oder der Antikörper mit einem Marker versehen wird.

17. Verfahren nach Anspruch 16, **dadurch gekennzeichnet, dass** der Marker ausgewählt ist aus der Gruppe bestehend aus radioaktiven Markern, farbigen Markern, enzymatischen Markern und magnetischen Markern.

18. Verfahren nach Anspruch 16 oder 17, **dadurch gekennzeichnet, dass** der Marker nach dem Binden eines Antikörpers eine Eigenschaftsänderung zeigt.

19. Kit zum Detektieren von *Enterococcus faecalis-* und/oder *Enterococcus faecium-*Antikörpern in einer Probe umfassend ein Antigen nach einem der Ansprüche 1 bis 7.

20. Kit zum Detektieren von *Enterococcus faecalis-* und/oder *Enterococcus faecium-*Antigenen in einer Probe umfassend einen monoklonalen Antikörper nach Anspruch 8, optional gebunden an einen Träger.

21. Kit nach Anspruch 19 oder 20, **dadurch gekennzeichnet, dass** das Antigen bzw. der Antikörper mit einem, Marker markiert ist.

22. Kit nach Anspruch 21, **dadurch gekennzeichnet, dass** der Marker ein radioaktiver Marker, ein farbiger Marker, ein enzymatischer Marker oder ein magnetischer Marker ist.

23. Kit nach einem der Ansprüche 19 bis 22, **dadurch gekennzeichnet, dass** das Antigen bzw. der Antikörper mit einer Matrix, bevorzugt mit einer festen Matrix, verbunden ist.

24. Verwendung eines Antigens gemäss Anspruch 1 bis 7 zur Detektion oder nicht-therapeutischen Herstellung von spezifischen *Enterococcus faecalis-* und/oder ***Enterococcus faecium*-Antikörpem** und/oder Antikörperfragmenten, insbesondere Fab oder scFv.

25. Verwendung nach Anspruch 24, wobei die hergestellten *Enterococcus faecalis-* und/oder *Enterococcus faecium-Antikörper* und/oder Antikörperfragmente zur passiven Immuntherapie oder zur Prophylaxe gegen Enterokokkenantigene vorgesehen sind.

26. Rekombinanter Antikörper **dadurch gekennzeichnet, dass** er strukturell dem Antikörper gemäss Anspruch 8 entspricht, oder ein rekombinantes Fab oder scFv Fragment davon.

27. Verfahren zur Herstellung eines Antikörpers gemäss Anspruch 8 oder eines Fab- oder scFv-Fragmentes davon in rekombinanter Form, umfassend das Klonieren einer DNA-Sequenz, die für diesen Antikörper oder dessen Fab- oder scFv-Fragment kodiert, in einen Expressionsvektor, das Transformieren einer Zelle, insbesondere einer *E. coli-*Zelle oder einer Hefezelle mit diesem Konstrukt und die Expression des rekombinanten Antikörpers oder eines Fab- oder scFv-Fragmentes davon.

## Claims

1. An *Enterococcus faecalis* and/or *Enterococcus faecium-antigen,* **characterized in that** it comprises a disaccharide structure consisting of a furanose Gal and a pyranose Glc, wherein the disaccharide structure has the following formula: 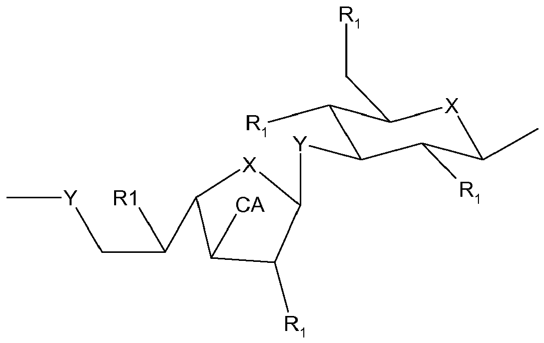
wherein R₁ is independently of one another selected from OH, OAc, OCₙHₘ, OFo, wherein Fo is formyl,
m = 2n + 1, and n is a natural number of 1 to 10,
X = O,
Y = O, and
CA independently of one another is a C₁-C₆-acyl or a hydroxylacyl residue.

2. The antigen according to claim 1, **characterized in that** R₁ = OH, X = O, Y = O and CA = lactyl.

3. The antigen according to claim 1 or 2, **characterized in that** it has a molecular weight of about 1000-200000 Da, more preferred of about 50000-150000 Da, particularly preferred of about 100000 Da.

4. The antigen according to one of claims 1 to 3, **characterized in that** per antigen the unit occurs at least 5 times, preferably at least 10 times, more preferred at least 100 times, particularly preferred at least 1000 times.

5. The antigen according to one of claims 1 to 4, **characterized in that** it is bound to a carrier.

6. The antigen according to claim 5, **characterized in that** the bond to the carrier is of covalent nature.

7. The antigen according to claim 5 or 6, **characterized in that** the carrier is an immune carrier.

8. A monoclonal antibody that is specific against an antigen according to one of claims 1 to 7.

9. A composition, comprising an antigen according to one of claims 1 to 7 or an antibody according to claim 8.

10. The composition according to claim 9, comprising a pharmaceutically acceptable carrier.

11. The composition according to claim 9 or 10, **characterized in that** it is a vaccine against *Enterococcus faecalis* and/or *Enterococcus faecium.*

12. The composition according to one of claims 9 to 11, **characterized in that** it is a pharmaceutical composition.

13. A method for detecting *of Enterococcus faecalis* and/or *Enterococcus faecium* antibodies, comprising
- contacting of antigens according to claim 1-7 with a sample to be tested for *Enterococcus faecalis* and/or *Enterococcus faecium* antibodies, and
- detecting of antibody-antigen or antibody-antigen conjugate-complexes,
wherein the presence of such complexes indicates the presence *of Enterococcus faecalis* and/or *Enterococcus faecium* antibodies in the sample.

14. A method for detecting *of Enterococcus faecalis* and/or *Enterococcus faecium* antigens, comprising
- contacting of monoclonal antibodies according to claim 8, optionally immobilized on a carrier, with a sample to be tested for *Enterococcus faecalis* and/or *Enterococcus faecium* antigens, and
- detecting of antigen-antibody or antibody conjugate-antigen complexes,
wherein the presence of such complexes indicates the presence *of Enterococcus faecalis* and/or *Enterococcus faecium* antigens in the sample.

15. The method according to claim 13 or 14, **characterized in that** the antigens or the antibodies are immobilized on a matrix, and in particular are immobilized on a solid matrix, preferably on a microtiter plate.

16. The method according to claims 13 to 15, **characterized in that** the antigen or the antibody is provided with a marker.

17. The method according to claim 16, **characterized in that** the marker is selected from the group consisting of radioactive markers, colored markers, enzymatic markers and magnetic markers.

18. The method according to claim 16 or 17, **characterized in that** the marker exhibits change of a characteristic after binding of an antibody.

19. A kit for detecting of *Enterococcus faecalis* and/or *Enterococcus faecium* antibodies in a sample, comprising an antigen according to one of claims 1 to 7.

20. A kit for detecting of *Enterococcus faecalis* and/or *Enterococcus faecium* antigens in a sample, comprising a monoclonal antibody according to claim 8, optionally bound to a carrier.

21. The kit according to claim 19 or 20, **characterized in that** the antigen or the antibody is labeled with a marker.

22. The kit according to claim 21, **characterized in that** the marker is a radioactive marker, a colored marker, an enzymatic marker or a magnetic marker

23. The kit according to one of claims 19 to 22, **characterized in that** the antigen or the antibody is bound to a matrix, preferably to a solid matrix.

24. Use of an antigen according to claim 1 to 7 for detecting or producing of specific *Enterococcus faecalis* and/or *Enterococcus faecium* antibodies and/or antibody fragments, in particular Fab or scFv.

25. The use according to claim 24, wherein the *Enterococcus faecalis* and/or *Enterococcus faecium* antibodies and/or antibody fragments as produced are provided for the passive immunotherapy or the prophylaxis against enterococcal antigens.

26. A recombinant antibody, **characterized in that** it structurally corresponds to the antibody according to claim 8, or a recombinant Fab or scFv fragment thereof.

27. A method for producing an antibody according to claim 8 or a Fab or scFv fragment thereof in recombinant form, comprising cloning of a DNA-sequence encoding for said antibody or its Fab or scFv fragment, into an expression vector, transforming of a cell, in particular of an *E. coli* cell or a yeast cell with this construct, and expressing of the recombinant antibody or the Fab or scFv fragment thereof.

## Revendications

1. Antigène *d'Enterococcus faecalis* et/ou *d'Enterococcus faecium,* **caractérisé en ce qu'**il comprend une structure disaccharidique constituée d'un furanose Gal et d'un pyranose Glc, dans lequel la structure disaccharidique a la formule suivante : 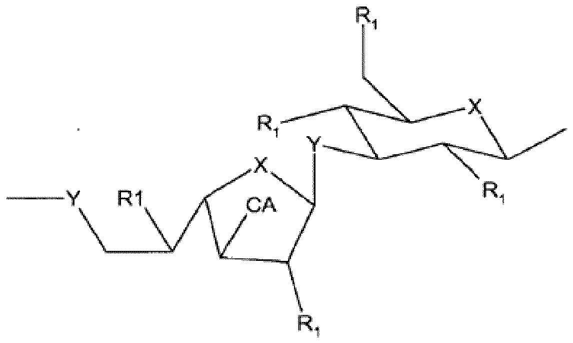
où R₁ est, indépendamment l'un de l'autre, choisi parmi OH, OAc, OCₙHₘ, OFo,
où Fo représente un groupement formyle,
m = 2n + 1, et n représente un nombre naturel allant de 1 à 10,
X = O,
Y = O, et
CA, indépendamment l'un de l'autre, représente un résidu acyle en C₁ à C₆ ou un résidu hydroxylacyle.

2. Antigène selon la revendication 1, **caractérisé en ce que** R₁ = OH, X = O, Y = O et CA = un groupement lactyle.

3. Antigène selon la revendication 1 ou 2, **caractérisé en ce qu'**il a un poids moléculaire allant d'environ 1000 à 200000 Da, de manière plus préférée allant d'environ 50000 à 150000 Da, de manière particulièrement préférée d'environ 100000 Da.

4. Antigène selon l'une des revendications 1 à 3, **caractérisé en ce que** par antigène l'unité apparaît au moins 5 fois, de préférence au moins 10 fois, de manière plus préférée au moins 100 fois, de manière particulièrement préférée au moins 1000 fois.

5. Antigène selon l'une des revendications 1 à 4, **caractérisé en ce qu'**il est lié à un support.

6. Antigène selon la revendication 5, **caractérisé en ce que** la liaison au support est de nature covalente.

7. Antigène selon la revendication 5 ou 6, **caractérisé en ce que** le support est un support immun.

8. Anticorps monoclonal qui est spécifique contre un antigène selon l'une des revendications 1 à 7.

9. Composition, comprenant un antigène selon l'une des revendications 1 à 7 ou un anticorps selon la revendication 8.

10. Composition selon la revendication 9, comprenant un support pharmaceutiquement acceptable.

11. Composition selon la revendication 9 ou 10, **caractérisée en ce qu'**elle représente un vaccin contre *Enterococcus faecalis* et/ou *Enterococcus faecium.*

12. Composition selon l'une des revendications 9 à 11, **caractérisée en ce qu'**elle est une composition pharmaceutique.

13. Procédé de détection d'anticorps *d'Enterococcus faecalis* et/ou *d'Enterococcus faecium,* comprenant le fait
de mettre en contact des antigènes selon les revendications 1 à 7 avec un échantillon à analyser pour rechercher des anticorps *d'Enterococcus faecalis* et/ou *d'Enterococcus faecium,* et
de détecter des complexes d'anticorps-antigène ou d'anticorps-antigène conjugué,
où la présence de tels complexes indique la présence d'anticorps *d'Enterococcus faecalis* et/ou d'*Enterococcus faecium* dans l'échantillon.

14. Procédé de détection d'antigènes d'*Enterococcus faecalis* et/ou d'*Enterococcus faecium,* comprenant le fait
de mettre en contact des anticorps monoclonaux selon la revendication 8, facultativement immobilisés sur un support, avec un échantillon à analyser pour rechercher des antigènes *d'Enterococcus faeca*lis et/ou d'*Enterococcus faecium,* et
de détecter des complexes d'antigène-anticorps ou d'anticorps conjugué-antigène,
où la présence de tels complexes indique la présence d'antigènes d'*Enterococcus faecalis* et/ou d'*Enterococcus faecium* dans l'échantillon.

15. Procédé selon la revendication 13 ou 14, **caractérisé en ce que** les antigènes ou les anticorps sont immobilisés sur une matrice, et en particulier sont immobilisés sur une matrice solide, de préférence sur une plaque de microtitration.

16. Procédé selon les revendications 13 à 15, **caractérisé en ce que** l'antigène ou l'anticorps est pourvu d'un marqueur.

17. Procédé selon la revendication 16, **caractérisé en ce que** le marqueur est choisi dans le groupe constitué de marqueurs radioactifs, de marqueurs colorés, de marqueurs enzymatiques et de marqueurs magnétiques.

18. Procédé selon la revendication 16 ou 17, **caractérisé en ce que** le marqueur présente un changement d'une caractéristique après la liaison d'un anticorps.

19. Kit de détection d'anticorps d'*Enterococcus faecalis* et/ou d'*Enterococcus faecium* dans un échantillon, comprenant un antigène selon l'une des revendications 1 à 7.

20. Kit de détection d'antigènes *d'Enterococcus faecalis* et/ou d'*Enterococcus faecium* dans un échantillon, comprenant un anticorps monoclonal selon la revendication 8, facultativement lié à un support.

21. Kit selon la revendication 19 ou 20, **caractérisé en ce que** l'antigène ou l'anticorps est marqué avec un marqueur.

22. Kit selon la revendication 21, **caractérisé en ce que** le marqueur est un marqueur radioactif, un marqueur coloré, un marqueur enzymatique ou un marqueur magnétique.

23. Kit selon l'une des revendications 19 à 22, **caractérisé en ce que** l'antigène ou l'anticorps est lié à une matrice, de préférence à une matrice solide.

24. Utilisation d'un antigène selon les revendications 1 à 7, pour la détection ou la production d'anticorps d'*Enterococcus faecalis* et/ou d'*Enterococcus faecium* spécifiques et/ou de fragments d'anticorps *d'Enterococcus faecalis* et/ou *d'Enterococcus faecium* spécifiques, en particulier Fab ou scFv.

25. Utilisation selon la revendication 24, dans laquelle les anticorps et/ou des fragments d'anticorps *d'Enterococcus faecalis* et/ou *d'Enterococcus faecium* tels que produits sont fournis pour l'immunothérapie passive ou la prophylaxie contre les antigènes d'entérocoques.

26. Anticorps recombinant, **caractérisé en ce qu'**il correspond d'un point de vue structure à l'anticorps selon la revendication 8, ou un fragment Fab ou scFv recombinant de celui-ci.

27. Procédé de production d'un anticorps selon la revendication 8 ou d'un fragment Fab ou scFv de celui-ci sous forme recombinante, comprenant le fait de cloner une séquence d'ADN codant pour ledit anticorps ou son fragment Fab ou scFv, dans un vecteur d'expression, de transformer une cellule, en particulier une cellule d'E. coli ou une cellule de levure avec cette construction, et d'exprimer l'anticorps recombinant ou le fragment Fab ou scFv de celui-ci.
